# EUROPEAN PATENT SPECIFICATION

(11) **EP 4 201 437 B1**
(45) Date of publication and mention of the grant of the patent: **15.05.2024**
(21) Application number: 22211752.5
(22) Date of filing: 06.12.2022
(51) Int. Cl.: A61L 24/08, A61L 26/00

(54) **HEMOSTATIC MATERIAL COMPOSITION**
HÄMOSTATISCHE MATERIALZUSAMMENSETZUNG
COMPOSITION DE MATÉRIAU HÉMOSTATIQUE

(30) Priority: 21.12.2021 JP 2021207605
(43) Date of publication of application: 28.06.2023
(73) Proprietor: FUJIFILM Corporation, Tokyo 106-8620 (JP)
(72) Inventor: KURAMOTO, Mamoru, Ashigarakami-gun, Kanagawa, 258-8538 (JP); YOSHITANI, Toshihide, Ashigarakami-gun, Kanagawa, 258-8538 (JP)
(74) Representative: Klunker IP Patentanwälte PartG mbB

(56) References cited:
- JP-B2- 6 195 568
- US-A1- 2006 178 339

## Description

### BACKGROUND OF THE INVENTION

### 1. Field of the Invention

The present disclosure relates to a hemostatic material composition.

### 2. Description of the Related Art

Uncontrollable bleeding is still the main cause of death of traumatic or surgical injuries. Accordingly, the development of an effective therapeutic approach for controlling bleeding is of vital clinical and social importance.

As hemostatic materials that are used in a surgical operation, fibrin glue, a non-woven fabric of oxidized cellulose, a collagen sponge, fine starch particles, a thrombin-containing gelatin paste, and the like have been known so far, and these are used in various forms.

Among them, FLOSEAL (registered trade name, manufactured by Baxter International Inc.) and SURGIFLO (manufactured by Ethicon, Inc.) are widely known as thrombin-containing gelatin pastes.

Thrombin activates fibrinogen contained in the blood to form a fibrin network, and the fibrin network has an action of sealing a bleeding part by forming a blood clot together with blood cells.

Although thrombin is purified from the human blood that has been properly sorted out through a step of inactivating viruses and a step of removing them, there is a problem that the risk of transmission of an infectious disease cannot be completely eliminated.

As a result, there is a demand for the development of a hemostatic material having excellent safety.

JP6195568B discloses a hemostatic material composition containing a polyethylene glycol having an N-hydroxysuccinimide ester group, a polyethylene glycol which is a hydrophilic solvent and containing crosslinked gelatin particles.

### SUMMARY OF THE INVENTION

The hemostatic material composition disclosed in JP6195568B is a viscous composition in which a polyethylene glycol having an N-hydroxysuccinimide ester group is dissolved in a polyethylene glycol which is a solvent. However, in a case where such a composition is viscous, the inventors of the present invention recently found newly a problem that there is room for improvement in hemostatic properties since not only it is difficult to apply a hemostatic composition to the bleeding part, but also it tends to be difficult to mix the composition with blood, and a polyethylene glycol having an N-hydroxysuccinimide ester group in a state of being dissolved tends to have low reactivity with an amino group or the like contained in proteins in the blood.

An object to be achieved by one aspect of the present disclosure is to provide a hemostatic material composition which is excellent in safety and hemostatic properties.

Specific means for solving the object are as follows.
<1> A hemostatic material composition comprising:
   a polysaccharide having a reactive group that reacts with a protein to form a crosslinking structure; and
   a hydrophobic solvent.
<2> The hemostatic material composition according to <1>, in which the reactive group is one or more groups selected from the group consisting of -CON(COCH₂)₂, an N-hydroxysuccinimide ester group, an imide ester group, an aldehyde group, a carboxy group, an isocyanate group, a maleimide group, and a haloacetyl group.
<3> The hemostatic material composition according to <1> or <2>, in which the reactive group is an N-hydroxysuccinimide ester group.
<4> The hemostatic material composition according to any one of <1> to <3>, in which the polysaccharide includes a structure derived from one or more polysaccharides selected from the group consisting of cellulose, dextran, dextrin, pullulan, hyaluronic acid, alginic acid, xanthan gum, chitin, chitosan, and a modified product thereof.
<5> The hemostatic material composition according to any one of <1> to <4>, in which the polysaccharide contains a constitutional unit derived from a monosaccharide having the reactive group, and a content of a constitutional unit derived from the monosaccharide having the reactive group is 1% by mole to 40% by mole with respect to 100% by mole of the polysaccharide.
<6> The hemostatic material composition according to <5>, in which the constitutional unit derived from the monosaccharide having the reactive group is represented by General Formula (1). 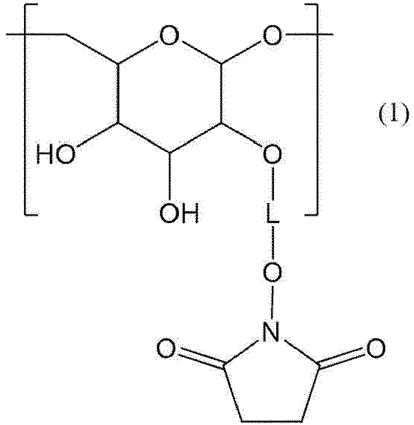 In General Formula (1), L is a group represented by the following formula. 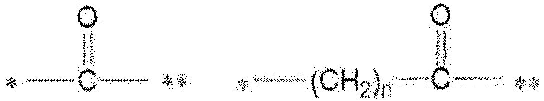 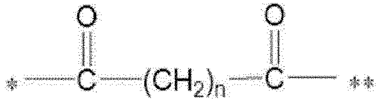 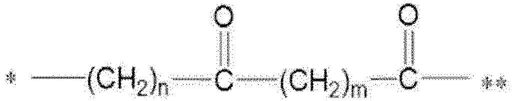 In the formula, n and m represent an integer of 1 to 3, * represents a site linked to an oxygen atom on a tetrahydrofuran side, and ** represents a site linked to an oxygen atom on an N-hydroxysuccinimide ester group side.
<7> The hemostatic material composition according to any one of <1> to <6>, in which a weight-average molecular weight of the polysaccharide is 2,000 to 2,500,000.
<8> The hemostatic material composition according to any one of <1> to <7>, in which the hydrophobic solvent is one or more selected from the group consisting of a vegetable oil, a paraffin-based solvent, an olefin-based solvent, a silicone oil-based solvent, a fatty acid ester-based solvent, an alkyl halide-based solvent, and a terpene-based solvent.
<9> The hemostatic material composition according to any one of <1> to <8>, in which a kinematic viscosity of the hydrophobic solvent at 25°C is 1 mm²/sec to 1,000 mm²/sec.
<10> The hemostatic material composition according to any one of <1> to <9>, in which a content of the hydrophobic solvent is 30% by mass to 70% by mass with respect to a total mass of the hemostatic material composition.
<11> The hemostatic material composition according to any one of <1> to <10>, further comprising a crosslinked particle.
<12> The hemostatic material composition according to <11>, in which the crosslinked particle contains one or more compounds selected from the group consisting of a crosslinked protein and a crosslinked polysaccharide.
<13> The hemostatic material composition according to <12>, in which the crosslinked particle contains the crosslinked polysaccharide, and the crosslinked polysaccharide is a crosslinked dextran.
<14> The hemostatic material composition according to <13>, in which the crosslinked dextran is a crosslinked dextran modified with an aminoalkyl group.
<15> The hemostatic material composition according to <14>, in which the aminoalkyl group is a diethylaminoethyl group.
<16> The hemostatic material composition according to any one of <11> to <15>, in which an average particle diameter of the crosslinked particles is 20 µm to 500 µm.

According to the present disclosure, it is possible to provide a hemostatic material composition which is excellent in safety and hemostatic properties.

### DESCRIPTION OF THE PREFERRED EMBODIMENTS

In the present disclosure, a numerical range described using "to" includes numerical values before and after "to" as a minimum value and a maximum value, respectively.

In the numerical ranges described stepwise in the present disclosure, the upper limit value or the lower limit value described in one numerical range may be replaced with the upper limit value or the lower limit value of the numerical range described stepwise in other stages. Further, in the numerical ranges described in the present disclosure, the upper limit value or the lower limit value of the numerical range may be replaced with the value shown in Examples.

In the present disclosure, the "hydrophobic solvent" means a solvent which has low miscibility with water, where 20 g or more of the solvent is not dissolved in 1 L of ion exchange water (pH 6 to 8) at 20°C, and does not form a uniform aqueous solution, that is, it refers to a solvent having a solubility in water of less than 20 g/L. In addition, in the present disclosure, the solvent having a solubility of 20 g/L or more is referred to as a hydrophilic solvent.

Here, the pH of the ion exchange water indicates a value measured at 20°C within 3 minutes after the ion exchange water comes into contact with air.

### Hemostatic material composition

A hemostatic material composition according to the present disclosure contains a polysaccharide (hereinafter, also referred to as a "specific polysaccharide") having a reactive group (hereinafter, also referred to as a "specific reactive group") that reacts with a protein to form a crosslinking structure, and a hydrophobic solvent.

The hemostatic material composition according to the present disclosure is excellent in safety and hemostatic properties. The reason why the above effect is obtained is presumed as follows, which is not limited thereto.

The hemostatic material composition according to the present disclosure contains a polysaccharide having a specific reactive group, and the specific reactive group reacts with an amino group or the like contained in a protein in the blood to form a crosslinking structure, whereby a coating film is formed and a hemostatic effect is exhibited. Therefore, it is not necessary to use thrombin, and thus it is presumed that the hemostatic material composition according to the present disclosure is excellent in safety.

In addition, since the hemostatic material composition according to the present disclosure contains a hydrophobic solvent, the specific polysaccharide is present in a state of being dispersed in the hydrophobic solvent, and the reactivity with an amino group or the like contained in a protein in the blood is excellent, it is presumed that the hemostatic material composition according to the present disclosure is excellent in hemostatic properties.

The state of the hemostatic material composition according to the present disclosure is not particularly limited; however, it is preferably a paste state from the viewpoint of ease of application to a living body.

### Specific polysaccharide

The specific polysaccharide has a specific reactive group that reacts with a protein to form a crosslinking structure.

The specific reactive group is not particularly limited as long as it is a group capable of reacting with an amino group or the like contained in a protein to form a crosslinking structure. However, from the viewpoint of improving hemostatic properties, it is preferably one or more groups selected from the group consisting of -CON(COCH₂)₂, an N-hydroxysuccinimide ester group (hereinafter, also referred to as an "NHS-ester group"), an imide ester group, an aldehyde group, a carboxy group, an isocyanate group, a maleimide group, and a haloacetyl group, and it is more preferably an NHS-ester group.

The specific polysaccharide may have two or more kinds of specific reactive groups. In addition, the hemostatic material composition according to the present disclosure may contain two or more kinds of specific polysaccharides.

It is noted that in the present disclosure, a polysaccharide that does not have a specific reactive group is also referred to as a non-specific polysaccharide.

The specific polysaccharide contains a constitutional unit derived from two or more monosaccharides.

From the viewpoint of improving hemostatic properties, the viewpoint of biocompatibility, and the like, the specific polysaccharide preferably includes a structure derived from one or more polysaccharides selected from the group consisting of cellulose, dextran, dextrin, pullulan, hyaluronic acid, alginic acid, xanthan gum, chitin, chitosan, and a modified product thereof, and it is more preferably include a structure derived from dextran.

The above-described structure contained in the polysaccharide is preferably a structure modified with a specific reactive group. In addition, the above-described structure contained in the polysaccharide may be carboxy-modified.

In the present disclosure, the modified product refers to a compound into which a functional group has been introduced by reacting cellulose or the like with a functional group such as a carboxy group.

From the viewpoint of improving hemostatic properties, the specific polysaccharide contains a constitutional unit (hereinafter, also referred to as a "specific constitutional unit") derived from a monosaccharide having a reactive group, and the content of the specific constitutional unit is preferably 1% by mole to 40% by mole, more preferably 3% by mole to 30% by mole, and still more preferably 5% by mole to 25% by mole, with respect to 100% by mole of the specific polysaccharide.

From the viewpoint of improving hemostatic properties, the viewpoint of biocompatibility, and the like, the specific constitutional unit is preferably a constitutional unit represented by General Formula (1).

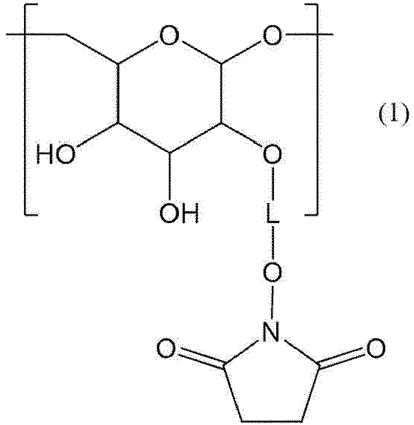

In General Formula (1), L is a group represented by the following formula.

In the following formula, n and m represent an integer of 1 to 3, * represents a site linked to an oxygen atom on a tetrahydrofuran side, and ** represents a site linked to an oxygen atom on an N-hydroxysuccinimide ester group side.

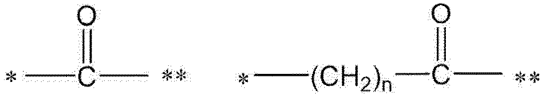

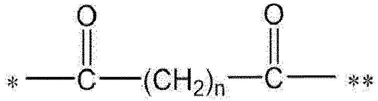

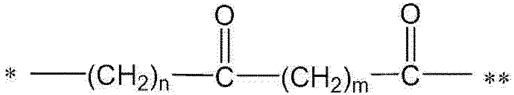

Among the above-described groups, from the viewpoint that the reaction rate of the reactive group is highest and the hemostatic properties are improved, L is preferably a group represented by the following formula.

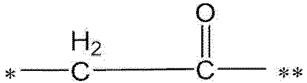

From the viewpoint of biocompatibility and the like, the specific polysaccharide preferably contains a constitutional unit represented by Formula (2) in addition to the constitutional unit represented by Formula (1).

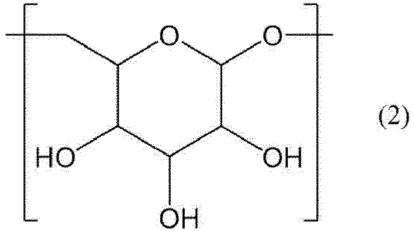

In a case where the specific polysaccharide contains the constitutional unit represented by Formula (2), the content of the constitutional unit represented by Formula (2) is preferably 40% by mole to 90% by mole and more preferably 45% by mole to 80% by mole with respect to 100% by mole of the specific polysaccharide.

The specific polysaccharide can contain a constitutional unit represented by Formula (3), whereby the pH at the time of dissolution in water can be decreased. Since the decomposition of the specific reactive group such as the N-hydroxysuccinimide ester group is suppressed in a solution having a low pH, it is possible to suppress a decomposition reaction of the composition or the like due to a small amount of water in the atmospheric air.

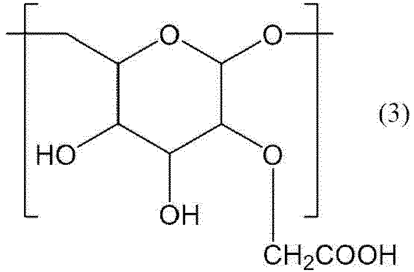

In a case where the specific polysaccharide contains the constitutional unit represented by Formula (3), the content of the constitutional unit represented by Formula (3) is preferably 5% by mole to 40% by mole and more preferably 8% by mole to 30% by mole with respect to 100% by mole of the specific polysaccharide.

The weight-average molecular weight (Mw) of the specific polysaccharide is preferably 2,000 to 2,500,000, more preferably 5,000 to 2,000,000, still more preferably 10,000 to 750,000, and particularly preferably 20,000 to 200,000.

In a case where the Mw of the specific polysaccharide is 2,000 or more, the specific reactive group contained in the specific polysaccharide reacts with a protein to form a good coating film in a case where a crosslinking structure is formed, and the hemostatic properties are further improved. In a case where the Mw of the specific polysaccharide is 2,500,000 or less, the specific polysaccharide can be easily synthesized, and the hemostatic material composition containing the specific polysaccharide is excellent in handleability.

The excess portion of the hemostatic material composition applied to the hemostasis site is preferably removed after the hemostasis is completed, and it is generally removed by the saline or the like. In a case where the Mw of the specific polysaccharide is 2,500,000 or less, the local viscosity in a case of being contact with the saline or the like is decreased, and thus the removability can be improved.

In the present disclosure, Mw is determined by dissolving a specimen in ultrapure water to a concentration of 0.1% by mass, carrying out filtration using a filter having a pore diameter of 0.45 µm, and subjecting the obtained filtrate to the gel permeation chromatography (GPC) measurement. The measurement conditions are as follows.

### Measurement condition

- Column: TSK gel G6000PW_{XL} + G4000PW_{XL} + G2500PW_{XL}
- Flow rate: 0.7 mL/min
- Column temperature: 40°C
- Injection volume: 100 µL
- Eluent: 100 mM NaNO₃ aq.
- Specimen concentration: 1,000 ppm
- Detection: Refractive index (RI) detector
- Analysis time: 60 minutes
- Standard specimen: Pullulan 5.8 k, 12.2 k, 23.7 k, 48.0 k, 100 k, 186 k, 380 k, 853 k

### (Shodex Standard P-82)

From the viewpoint of improving hemostatic properties, the content of the specific polysaccharide is preferably 1% by mass to 60% by mass, more preferably 5% by mass to 50% by mass, and still more preferably 15% by mass to 30% by mass, with respect to the total mass of the hemostatic material composition.

From the viewpoint of improving hemostatic properties and removability, the solubility of the specific polysaccharide is preferably 1% by mass or less and more preferably 0.1% by mass or less in 100 g of the hydrophobic solvent at 20°C. The lower limit of the solubility is not particularly limited and may be 0% by mass.

For example, the fact that the solubility is 1% by mass in 100 g of a hydrophobic solvent at 20°C means that the amount of the specific polysaccharide dissolved in 100 g of the hydrophobic solvent is 1 g.

### Hydrophobic solvent

From the viewpoint of improving hemostatic properties and removability, the hydrophobic solvent preferably has a solubility of less than 20 g/L, more preferably less than 5 g/L, and still more preferably less than 1 g/L in 1 L of ion exchange water at 20°C. The lower limit of the solubility is not particularly limited and may be 0 g/L.

The kind of the hydrophobic solvent is not particularly limited. From the viewpoint of improving hemostatic properties and the viewpoint of biocompatibility, the hydrophobic solvent is preferably one or more selected from the group consisting of a vegetable oil, a paraffin-based solvent, an olefin-based solvent, a silicone oil-based solvent, a fatty acid ester-based solvent, an alkyl halide-based solvent, and a terpene-based solvent, and it is more preferably a vegetable oil.

In the present disclosure, the "vegetable oil" is not particularly limited as long as it is an oil obtained by using a plant as a raw material, where it may be a derivative thereof, and a vegetable oil containing a fatty acid glyceride is preferable. Examples of the vegetable oil include a linseed oil, a wild sesame oil, a olive oil, a grapeseed oil, a corn oil, a coconut oil, a sesame oil, a rice bran oil, a soybean oil, a rapeseed oil, a palm oil, a sunflower oil, safflower oil, a cottonseed oil, a groundnut oil, an avocado oil, an almond oil, an argan oil, a cacao oil, a shea butter, a tea seed oil, a peanut oil, and a castor oil.

Examples of the paraffin-based solvent include octane, decane, dodecane, and tridecane.

Examples of the olefin-based solvent include decene and dodecene.

Examples of the silicone oil-based solvent include a dimethyl silicone oil, a methylphenyl silicone oil, and a cyclic dimethyl silicone oil.

Examples of the fatty acid ester-based solvent include methyl laurate, methyl palmitate, methyl stearate, methyl oleate, palm methyl oleate, caprylic acid 2-ethylhexyl ester, glycerin monostearate, glycerin monobehenate, glycerin mono-12-hydroxystearate, glycerin monooleate, glycerin monocaprylate, glycerin monocaprate, glycerin monolaurate, an alkyl oleate (ethyl oleate, methyl oleate, or the like), triglyceride oleate, an alkyl linoleate (methyl linoleate, ethyl linoleate, or the like), linoleic acid triglyceride, and dioleic acid glyceride.

Examples of the alkyl halide-based solvent include methylene chloride and 1,1,1-trichloroethane.

Examples of the terpene-based solvent include D-limonene.

The hemostatic material composition according to the present disclosure may contain two or more kinds of hydrophobic solvents.

The molecular weight of the hydrophobic solvent is preferably 300 to 1,500.

Due to being used for medical use application, the hydrophobic solvent may become hot instantaneously by the high-frequency treatment or the like. As a result, from the viewpoint of safety assuming an actual operation, the hydrophobic solvent is preferably non-volatile. The non-volatile solvent refers to a solvent having a boiling point of 50°C or higher, where the boiling point is preferably 100°C or higher and more preferably 200°C or higher.

From the viewpoint of improving the ease of application to a living body and the removability by washing with water from the living body, and suppressing the temporal liquid separation, the kinematic viscosity of the hydrophobic solvent at 25°C is preferably 1 mm²/sec to 1,000 mm²/sec, more preferably 5 mm²/sec to 200 mm²/sec, and still more preferably 20 mm²/sec to 100 mm²/sec.

In the present disclosure, the kinematic viscosity is measured in accordance with JIS K 2283 (2000).

From the viewpoint of improving hemostatic properties, the content of the hydrophobic solvent is preferably 25% by mass to 75% by mass, more preferably 30% by mass to 70% by mass, and still more preferably 40% by mass to 60% by mass, with respect to the total mass of the hemostatic material composition.

### Crosslinked particle

The hemostatic material composition according to the present disclosure may contain crosslinked particles. Regarding the hemostatic material composition, in a case where the hemostatic material composition containing crosslinked particles is mixed with blood, water in the blood is taken up by the crosslinked particles, the concentration of proteins in the blood is increased, the reactivity with the specific polysaccharide is improved, and the hemostatic properties are further improved. In the present disclosure, the crosslinked particle means a particle in which proteins or the like are crosslinked.

In addition, for the intended purpose of improving the hemostatic effect, the hemostatic material composition applied to the bleeding site may be pressed with gauze or the like, which has been impregnated with a saline solution or the like. In a case where the hemostatic material composition contains the crosslinked particles, it is possible to suppress the transfer of the hemostatic material composition to a gauze or the like, which is peeled off after pressing. This is presumed to be because the crosslinked particle takes up the saline or the like, with which gauze or the like has been impregnated, and swells depending on the degree of crosslinking, whereby the local viscosity on the contact surface between the gauze and the hemostatic material composition decreases.

From the viewpoint of improving the properties of taking up water and improving hemostatic properties, the crosslinked particle preferably contains a hydrophilic polymer.

Examples of the hydrophilic polymer include a protein and a polysaccharide. That is, from the viewpoint of improving the properties of taking up water and the hemostatic property, it is preferable that the crosslinked particle contains one or more compounds selected from the group consisting of a crosslinked protein and a crosslinked polysaccharide. In the present disclosure, the crosslinked protein refers to a crosslinked protein in which two or more proteins are crosslinked, and the crosslinked polysaccharide refers to a product in which two or more polysaccharides are crosslinked.

Examples of the protein include gelatin, collagen, albumin, hemoglobin, fibrinogen, fibrin, casein, elastin, and keratin.

Examples of the polysaccharide include cellulose, dextran, dextrin, pullulan, hyaluronic acid, alginic acid, xanthan gum, chitin, chitosan, and a modified product thereof.

The hemostatic material composition according to the present disclosure may contain two or more kinds of crosslinked particles.

From the viewpoint of improving the properties of taking up water and improving hemostatic properties and the viewpoint of biocompatibility, it is preferable that the crosslinked particle contains a crosslinked polysaccharide and the crosslinked polysaccharide is a crosslinked dextran.

From the viewpoint of improving the properties of taking up water, the crosslinked dextran is preferably modified with an aminoalkyl group. The nitrogen atom contained in the aminoalkyl group may be substituted with an alkyl group.

In a case where the hemostatic material composition according to the present disclosure contains particles of the crosslinked dextran modified with an aminoalkyl group and in a case where the hemostatic material composition according to the present disclosure is applied to a living body, the hemostatic property can be improved since the specific reactive group and the aminoalkyl group form a covalent bond, an ionic bond, or a hydrogen bond to improve the coating film hardness.

From the viewpoint of improving the properties of taking up water and improving hemostatic properties, the aminoalkyl group is preferably a diethylaminoethyl group.

From the viewpoint of improving the properties of taking up water and improving hemostatic properties, the viewpoint of suppressing the transfer of the hemostatic material composition to gauze, and the viewpoint of suppressing the invasion into blood vessels, and the like, the average particle diameter of the crosslinked particles is preferably 20 µm to 500 µm and more preferably 40 µm to 200 µm.

In the present disclosure, the measurement of the average particle diameter is determined by arithmetically averaging the particle diameters of 50 particles randomly selected from an image of a scanning electron microscope (SEM). It is noted that the primary particle diameter is measured as the particle diameter of the particle.

From the viewpoint of improving the properties of taking up water and improving hemostatic properties, the viewpoint of suppressing the transfer of the hemostatic material composition to gauze, and the viewpoint of suppressing the invasion into blood vessels, and the like, the content of the crosslinked particle is preferably 1% by mass to 50% by mass, more preferably 8% by mass to 40% by mass, and still more preferably 20% by mass to 40% by mass, with respect to the total mass of the hemostatic material composition.

### Component other than specific polysaccharide, hydrophobic solvent, and crosslinked particle

The hemostatic material composition according to the present disclosure may contain a component other than the specific polysaccharide, and the hydrophobic solvent, and the crosslinked particle, and examples thereof include a stabilizer such as ascorbic acid, an antibiotic, a vasoconstrictor, a pigment, a growth factor, a proliferation factor, a bone morphogenetic protein, an analgesic agent, an anti-inflammatory agent, and an antifibrinolytic agent such as tranexamic acid.

### Examples

Hereinafter, the above-described embodiment will be specifically described with reference to Examples; however, the above-described embodiment is not limited to these Examples. It is noted that the unit of the numerical values in Tables 1 to 3 is % by mass unless otherwise specified.

### Synthesis Example 1

10 g of a sodium carboxymethyl dextran A (manufactured by Meito Sangyo Co., Ltd., CMD-D40, Mw: 40,000) was dissolved in 100 g of distilled water, and 13 g of a 10% by mass hydrochloric acid aqueous solution was added thereto. The obtained solution was reprecipitated in 1,600 mL of methanol, and the filtrate was washed with methanol and then subjected to vacuum drying at room temperature (25°C) for 24 hours to obtain 10.44 g of a carboxymethyl dextran X.

Next, 10 g of the carboxymethyl dextran X was dissolved in 100 g of dimethylacetamide (DMAc), and 3.78 g of N-hydroxysuccinimide (manufactured by Fujifilm Wako Pure Chemical Corporation) and 6,30 g of 1-ethyl-3-(3-dimethylaminopropyl) carbodiimide hydrochloride (EDC hydrochloride, manufactured by FUJIFILM Wako Pure Chemical Corporation) were added thereto, and stirring was carried out at 40°C for 4 hours.

The obtained solution was reprecipitated in 350 mL of methanol, the filtrate was washed with methanol and acetone and subjected to vacuum drying at room temperature (25°C) for 24 hours to obtain 10.6 g of carboxymethyl dextran A having N-hydroxysuccinimide ester group (hereinafter, also referred to as an "NHS-carboxymethyl dextran"). It is noted that the NHS-carboxymethyl dextran A is composed of a constitutional unit represented by General Formula (1), a constitutional unit represented by Formula (2), and a constitutional unit represented by Formula (3).

10 mg of the NHS-carboxymethyl dextran A was dissolved in 1 g of heavy water, heated to 40°C, and hydrolyzed for 24 hours.

A hydrolyzate of the NHS-carboxymethyl dextran A was obtained, and in the spectrum obtained using a nuclear magnetic resonance apparatus (manufactured by BRUKER, 400 MHz NMR, number of integrations: 16 times), the ratio of the liberated proton (2.67 ppm to 2.74 ppm) to the proton (4.82 ppm to 5.47 ppm)) at the 1-position of the glucopyranose ring was determined to calculate the content of the constitutional unit (the constitutional unit represented by General Formula (1)) derived from a monosaccharide having an N-hydroxysuccinimide ester group with respect to 100% by mole of the NHS-carboxymethyl dextran A. As a result, it was 14.8% by mole.

Similarly, the contents of the constitutional unit represented by Formula (2) and the constitutional unit represented by Formula (3) were calculated and were found to be 70% by mole and 15.2% by mole, respectively.

In addition, the Mw of the NHS-carboxymethyl dextran A was 45,000.

### Synthesis Example 2

19.6 g of sodium hydroxide was dissolved in 106 g of distilled water, and 10 g of pullulan (manufactured by Tokyo Chemical Industry Co., Ltd.) was gradually added thereto a little bit at a time to be dissolved, and stirring was carried out for 90 minutes.

After the stirring, an aqueous solution of chloroacetic acid (an aqueous solution obtained by dissolving 7 g of chloroacetic acid in 63 g of distilled water) was added thereto, and a reaction was allowed to proceed at 60°C for 6 hours.

After cooling to room temperature (25°C), 65 g of a 20% by mass hydrochloric acid aqueous solution was added thereto, and stirring was carried out for 2 hours. The obtained solution was reprecipitated in 2,500 mL of methanol, and the filtrate was washed with methanol and then subjected to vacuum drying at room temperature (25°C) for 24 hours to obtain 10.85 g of carboxymethyl pullulan. An NHS-carboxymethyl pullulan was obtained in the same manner as in Synthesis Example 1 except that the carboxymethyl dextran X was changed to carboxymethyl pullulan and the adding amounts of N-hydroxysuccinimide and EDC hydrochloride were changed to 1.69 g and 2.81 g, respectively. It is noted that the NHS-carboxymethyl pullulan is composed of a constitutional unit represented by General Formula (1), a constitutional unit represented by Formula (2), and a constitutional unit represented by Formula (3).

In addition, as a result of calculating the content of the constitutional unit (the constitutional unit represented by General Formula (1)) derived from a monosaccharide having an N-hydroxysuccinimide ester group with respect to 100% by mole of the NHS-carboxymethyl pullulan, it was 6.3% by mole.

Similarly, the contents of the constitutional unit represented by Formula (2) and the constitutional unit represented by Formula (3) were calculated and were found to be 70% by mole and 23.7% by mole, respectively.

In addition, the Mw of the NHS-carboxymethyl pullulan was 12,000.

### Synthesis Example 3

19.6 g of sodium hydroxide was dissolved in 106 g of distilled water, and 10 g of dextrin (manufactured by Sanwa Starch Co., Ltd., Sandek (registered trade name) # 100, Mw: 17,000) was gradually added a little bit at a time to be dissolved, and stirring was carried out for 90 minutes.

After the stirring, an aqueous solution of chloroacetic acid (an aqueous solution obtained by dissolving 7 g of chloroacetic acid in 63 g of distilled water) was added thereto, and a reaction was allowed to proceed at 60°C for 6 hours.

After cooling to room temperature (25°C), 65 g of a 20% by mass hydrochloric acid aqueous solution was added thereto, and stirring was carried out for 2 hours. The obtained solution was reprecipitated in 2,500 mL of methanol, and the filtrate was washed with methanol and then subjected to vacuum drying at room temperature (25°C) for 24 hours to obtain 10.25 g of carboxymethyl dextrin. An NHS-carboxymethyl dextrin was obtained in the same manner as in Synthesis Example 1 except that the carboxymethyl dextran X was changed to carboxymethyl dextrin and the adding amounts of N-hydroxysuccinimide and EDC hydrochloride were changed to 3.31 g and 5.51 g, respectively. It is noted that the NHS-carboxymethyl dextrin is composed of a constitutional unit represented by General Formula (1), a constitutional unit represented by Formula (2), and a constitutional unit represented by Formula (3).

In addition, as a result of calculating the content of the constitutional unit (the constitutional unit represented by General Formula (1)) derived from a monosaccharide having an N-hydroxysuccinimide ester group with respect to 100% by mole of the NHS-carboxymethyl dextrin, it was 10% by mole.

Similarly, the contents of the constitutional unit represented by Formula (2) and the constitutional unit represented by Formula (3) were calculated and were found to be 70% by mole and 20% by mole, respectively.

In addition, the Mw of NHS-carboxymethyl dextrin was 18,000.

### Synthesis Example 4

10 g of the carboxymethyl dextran X synthesized in Synthesis Example 1 was dissolved in 100 g of DMAc, and each of 5.67 g of N-hydroxysuccinimide (manufactured by Fujifilm Wako Pure Chemical Corporation) and 9.45 g of EDC hydrochloride (manufactured by Fujifilm Wako Pure Chemical Corporation) was added thereto, and stirring was carried out at 40°C for 4 hours.

The obtained solution was reprecipitated in 350 mL of methanol, and the filtrate was washed with methanol and acetone and then subjected to vacuum drying at room temperature (25°C) for 24 hours to obtain 11.2 g of an NHS-carboxymethyl dextran B. It is noted that the NHS-carboxymethyl dextran B is composed of a constitutional unit represented by General Formula (1), a constitutional unit represented by Formula (2), and a constitutional unit represented by Formula (3).

In addition, as a result of calculating the content of the constitutional unit (the constitutional unit represented by General Formula (1)) derived from a monosaccharide having an N-hydroxysuccinimide ester group with respect to 100% by mole of the NHS-carboxymethyl dextran B, it was 21% by mole.

Similarly, the contents of the constitutional unit represented by Formula (2) and the constitutional unit represented by Formula (3) were calculated and were found to be 70% by mole and 9% by mole, respectively.

In addition, the Mw of NHS-carboxymethyl dextran B was 47,000.

### Synthesis Example 5

10 g of the carboxymethyl dextran X synthesized in Synthesis Example 1 was dissolved in 100 g of DMAc, and each of 1.32 g of N-hydroxysuccinimide (manufactured by Fujifilm Wako Pure Chemical Corporation) and 2.20 g of EDC hydrochloride (manufactured by Fujifilm Wako Pure Chemical Corporation) was added thereto, and stirring was carried out at 40°C for 4 hours.

The obtained solution was reprecipitated in 350 mL of methanol, and the filtrate was washed with methanol and acetone and then subjected to vacuum drying at room temperature (25°C) for 24 hours to obtain 10.1 g of an NHS-carboxymethyl dextran C. It is noted that the NHS-carboxymethyl dextran C is composed of a constitutional unit represented by General Formula (1), a constitutional unit represented by Formula (2), and a constitutional unit represented by Formula (3).

In addition, as a result of calculating the content of the constitutional unit (the constitutional unit represented by General Formula (1)) derived from a monosaccharide having an N-hydroxysuccinimide ester group with respect to 100% by mole of the NHS-carboxymethyl dextran C, it was 2.2% by mole.

Similarly, the contents of the constitutional unit represented by Formula (2) and the constitutional unit represented by Formula (3) were calculated and were found to be 70% by mole and 27.8% by mole, respectively.

In addition, the Mw of NHS-carboxymethyl dextran C was 44,000.

### Synthesis Example 6

An NHS-carboxymethyl dextran D was obtained in the same manner as in Synthesis Example 1 except that the sodium carboxymethyl dextran A was changed to a sodium carboxymethyl dextran B (manufactured by Meito Sangyo Co., Ltd., CMD-500, Mw: 500,000). It is noted that the NHS-carboxymethyl dextran D is composed of a constitutional unit represented by General Formula (1), a constitutional unit represented by Formula (2), and a constitutional unit represented by Formula (3).

In addition, as a result of calculating the content of the constitutional unit (the constitutional unit represented by General Formula (1)) derived from a monosaccharide having an N-hydroxysuccinimide ester group with respect to 100% by mole of the NHS-carboxymethyl dextran D, it was 15% by mole.

Similarly, the contents of the constitutional unit represented by Formula (2) and the constitutional unit represented by Formula (3) were calculated and were found to be 70% by mole and 15% by mole, respectively.

In addition, the Mw of NHS-carboxymethyl dextran D was 530,000.

### Synthesis Example 7

An NHS-carboxymethyl dextran E was obtained in the same manner as in Synthesis Example 1 except that the sodium carboxymethyl dextran A was changed to a sodium carboxymethyl dextran C (manufactured by Meito Sangyo Co., Ltd., CMD-L, Mw: 10,000). It is noted that the NHS-carboxymethyl dextran G is composed of a constitutional unit represented by General Formula (1), a constitutional unit represented by Formula (2), and a constitutional unit represented by Formula (3).

In addition, as a result of calculating the content of the constitutional unit (the constitutional unit represented by General Formula (1)) derived from a monosaccharide having an N-hydroxysuccinimide ester group with respect to 100% by mole of the NHS-carboxymethyl dextran E, it was 15% by mole.

Similarly, the contents of the constitutional unit represented by Formula (2) and the constitutional unit represented by Formula (3) were calculated and were found to be 70% by mole and 15% by mole, respectively.

In addition, the Mw of NHS-carboxymethyl dextran E was 11,000.

### Synthesis Example 8

5 g of diphenylmethane diisocyanate (manufactured by Fujifilm Wako Pure Chemical Corporation) was dissolved in a mixed solution of 90 g of toluene (manufactured by Fujifilm Wako Pure Chemical Corporation) and 5 g of diphenylmethane diisocyanate (manufactured by Fujifilm Wako Pure Chemical Corporation), and 5 g of chitosan (manufactured by GENERAL SCIENCE CORPORATION, Mw: 15,000, powder-shaped, degree of deacetylation: 84%) was dispersed therein.

The obtained dispersion liquid was stirred at 100°C for 24 hours, cooled to room temperature (25°C) and filtered, and then the obtained powder washed with toluene and acetone and subjected to air drying to obtain a powder of isocyanated chitosan.

In addition, the Mw of the isocyanated chitosan was 15,000.

### Examples 1 to 24 and Comparative Examples 1 to 5

Components shown in Tables 1 to 3 were mixed to produce each paste-shaped hemostatic material composition.

The details of the components shown in Tables 1 to 3 are as follows.

### Hydrophobic solvent

· Vegetable oil A: manufactured by Kenei Pharmaceutical Co., Ltd., an olive oil according to Japanese Pharmacopoeia, kinematic viscosity at 25°C: 72 mm²/sec, non-volatile
· Vegetable oil B: manufactured by KANEDA Co., Ltd., a soybean oil according to Japanese Pharmacopoeia, kinematic viscosity at 25°C: 60 mm²/sec, non-volatile
· Paraffin-based solvent: manufactured by Nichi-Iko Pharmaceutical Company, Limited, liquid paraffin NikP, kinematic viscosity at 25°C: 37 mm²/sec, non-volatile
· Silicone oil-based solvent A: manufactured by Shin-Etsu Chemical Co., Ltd., KF-96-10cs, kinematic viscosity at 25°C: 10 mm²/sec, non-volatile
· Silicone oil-based solvent B: manufactured by Shin-Etsu Chemical Co., Ltd., KF-96-500cs, kinematic viscosity at 25°C: 500 mm²/sec, non-volatile
· Fatty acid ester-based solvent: methyl oleate, manufactured by Kao Corporation, EXEPARL (registered trade name) M-OL, kinematic viscosity at 25°C: 5 mm²/sec, non-volatile

When 20 g of the above-described hydrophobic solvent was put into 1 L of ion exchange water (pH 7) at 20°C, it was confirmed that the solution is separated into two layers and is not an uniform aqueous solution, and the solubility in water is less than 20 g/L.

### Hydrophilic solvent

· Polyethylene glycol: manufactured by Fujifilm Wako Pure Chemical Corporation, PEG 200, kinematic viscosity at 25°C: 62 mm²/sec, non-volatile
· Polyethylene glycol/polypropylene glycol copolymer: manufactured by BASF SE, Pluronic L61, kinematic viscosity at 25°C: 300 mm²/sec, non-volatile

When 20 g of the above-described hydrophilic solvent was put into 1 L of ion exchange water (pH 7) at 20°C, it was confirmed that the solution is an uniform aqueous solution, and the solubility in water is 20 g/L or more.

### Crosslinked particle

· Crosslinked particle A: a particle of crosslinked dextran modified with a diethylaminoethyl group, manufactured by Cytiva, DEAE-Sephadex (registered trade name) A-50, average particle diameter: 40 µm to 100 µm
· Crosslinked particle B: a particle of crosslinked cellulose, manufactured by Merck KGaA, Parteck (registered trade name) CCS croscarmellose sodium, average particle diameter: 60 µm
· Crosslinked particle C: a particle of crosslinked starch sodium glycolate, manufactured by DFE Pharma, Primojel (registered trade name), average particle diameter: 50 µm
· Crosslinked particle D: a particle of crosslinked gelatin, prepared by a method described below, average particle diameter: 20 µm to 500 µm

High Grade gelatin (Mw: 60,000) manufactured by Nippi. Inc. was irradiated with γ-rays so that the absorbed dose was 50 kGy to obtain crosslinked gelatin
· Crosslinked particle E: a particle of crosslinked dextran, manufactured by Cytiva, Sephadex (registered trade name) A-50, average particle diameter: 40 µm to 120 µm
· Crosslinked particle F: a particle of crosslinked dextran, manufactured by Cytiva, QAE Sephadex (registered trade name) A-50 Chloride form, average particle diameter: 40 µm to 120 µm

### Non-crosslinked particle

· Non-crosslinked particle A: a particle of dextran modified with a diethylaminoethyl group, manufactured by Fujifilm Wako Pure Chemical Corporation, DEAE-dextran

### Evaluation of hemostatic properties

The abdomen of the pig was opened, and a bleeding wound part having a diameter of 8 mm and a depth of 2 mm was formed on the surface part of the liver with a biopsy trephine.

0.5 g of the hemostatic material composition produced in each of Examples 1 to 24 and Comparative Examples 1 to 5 was applied onto the bleeding wound part so that the bleeding wound part was covered, a coating film was allowed to be formed, and the bleeding wound part was pressed with gauze wetted with the saline.

The state of the bleeding wound part after 1 minute, 2 minutes, 5 minutes, and 10 minutes from the application of the hemostatic material composition was visually observed and evaluated based on the following evaluation standards, and the results were summarized in Table 1 to Table 3. It is noted that it was determined that the hemostatic properties are good in a case where the evaluation is 3 to 5.

### Evaluation standard

5: It could be confirmed that the hemostasis is achieved when observing the state of the bleeding wound part after 1 minute.
4: It could be confirmed that although the hemostasis is not achieved when observing the state of the bleeding wound part after 1 minute, the hemostasis is achieved when observing the state of the bleeding wound part after 2 minutes.
3: It could be confirmed that although the hemostasis is not achieved when observing the state of the bleeding wound part after 2 minutes, the hemostasis is achieved when observing the state of the bleeding wound part after 5 minutes.
2: It could be confirmed that although the hemostasis is not achieved when observing the state of the bleeding wound part after 2 minutes, the hemostasis is achieved when observing the state of the bleeding wound part after 5 minutes.
1: It could be confirmed that the hemostasis is not achieved even when observing the state of the bleeding wound part after 10 minutes.

### Evaluation of removability

The hemostatic material composition produced in each of Examples 1 to 24 and Comparative Examples 1 to 5 was applied onto a slide glass plate (2.5 cm × 7.5 cm) to a thickness of 2 mm.

The slide glass plate after the coating was gently immersed in 50 mL of the saline, taken out after 10 seconds and 1 minute, visually observed, evaluated based on the following evaluation standards, and summarized in Tables 1 to 3. It is noted that it was determined that the removability is good in a case where the evaluation is 3 to 5.

### Evaluation standard

5: The residual of the hemostatic material composition was not observed in the slide glass plate taken out 10 seconds after the start of the immersion.
4: Although the slight residual of the hemostatic material composition was observed on the slide glass plate taken out 10 seconds after the start of the immersion, the residual of the hemostatic material composition was not observed in the slide glass plate taken out 1 minute after the start of the immersion.
3: The slight residual of the hemostatic material composition was observed in any of the slide glass plates taken out 10 seconds and 1 minute after the start of the immersion.
2: The large residual of the hemostatic material composition was observed in any of the slide glass plates taken out 10 seconds and 1 minute after the start of the immersion.
1: It was confirmed that in any of the slide glass plates taken out 10 seconds and 1 minute after the start of the immersion, there are no signs that the hemostatic material composition has been removed, and almost the entire amount of the applied hemostatic material composition is remained.

### Evaluation of transfer suppressibility

0.5 g of the hemostatic material composition produced in each of Examples 1 to 24 and Comparative Examples 1 to 5 was applied onto the surface of the excised porcine liver, a coating film was allowed to be formed, and the bleeding wound part was pressed for 2 minutes with gauze wetted with the saline.

Then, the gauze was slowly peeled off, and the surface of the gauze and the surface of the excised liver were visually observed to carry out the evaluation based on the following evaluation standards, which are summarized in Table 1 to Table 3. It is noted that it was determined that the transfer suppressibility is good in a case where the evaluation is 3 to 5.

### Evaluation standard

5: No transfer of the hemostatic material composition to the gauze was observed, and no peeling of the coating film on the surface of the excised liver was observed.
4: Although the transfer of the hemostatic material composition to the gauze was slightly observed, no peeling of the coating film on the surface of the excised liver was observed.
3: The transfer of the hemostatic material composition to the gauze was slightly observed, and the peeling of the coating film on the surface of the excised liver was slightly observed.
2: The large transfer of the hemostatic material composition to the gauze was observed, and the large peeling of the coating film on the surface of the excised liver was observed.
1: The large transfer of the hemostatic material composition to the gauze was observed, and most of the coating film on the surface of the excised liver was peeled off.

**Table 1**

| | Kind | Content of specific structural unit with respect to 100% by mole of specific polysaccharide (% by mole) | Example 1 | Example 2 | Example 3 | Example 4 | Example 5 | Example 6 | Example 7 | Example 8 | Example 9 | Example 10 | Example 11 | Example 12 |
|---|---|---|---|---|---|---|---|---|---|---|---|---|---|---|
| Specific polysaccharide | NHS-carboxymethyl dextran A | 14.8% by mole | 20 | 20 | 20 | 20 | 20 | 20 | 50 | - | - | - | - | - |
| | NHS-carboxmethyl pullulan | 6.3% by mole | - | - | - | - | - | - | - | 20 | - | - | - | - |
| | NHS-carboxymethyl dextrin | 10% by mole | - | - | - | - | - | - | - | - | 20 | - | - | - |
| | NHS-carboxymethyl dextran B | 21% by mole | - | - | - | - | - | - | - | - | - | 20 | - | - |
| | NHS-carboxymethyl dextran C | 2.2% by mole | - | - | - | - | - | - | - | - | - | - | 20 | - |
| | 24HS-carboxymethyl dextran D | 15% by mole | - | - | - | - | - | - | - | - | - | - | - | 20 |
| | NHS-carboxymethyl dextran E | 15% by mole | - | - | - | - | - | - | - | - | - | - | - | - |
| | Isocyanated chitosan | - | - | - | - | - | - | - | - | - | - | - | - | - |
| Hydrophilic solvent | Kind | Solubility in ion exchange water at 20°C | | | | | | | | | | | | |
| | Vegetable oil A | Less than 20 g/L | 50 | 50 | 50 | 50 | 50 | 50 | 50 | 50 | 50 | 50 | 50 | 50 |
| | Vegetable oil B | Less than 20 g/L | - | - | - | - | - | - | - | - | - | - | - | - |
| | Paraffin-based solvent | Less than 20 g/L | - | - | - | - | - | - | - | - | - | - | - | - |
| | Silicone oil-based solvent A | Less than 20 g/L | - | - | - | - | - | - | - | - | - | - | - | - |
| | Silicone oil-based solvent B | Less than 20 g/L | - | - | - | - | - | - | - | - | - | - | - | - |
| | Fatty acid ester-based solvent | Less than 20 g/L | - | - | - | - | - | - | - | - | - | - | - | - |
| Crosslinked particle | Kind | Average particle diameter | | | | | | | | | | | | |
| | Crosslinked particle | 40 µm to 60 µm | 30 | - | - | - | - | - | - | 30 | 30 | 30 | 30 | 30 |
| | Crosslinked particle B | 60 µm | - - | 30 | - | - | - | - | - | - | - | - | - | - |
| | Crosslinked particle C | 50 µm | - | - | 30 | - | - | - | - | - | - | - | - | - |
| | Crosslinked particle D | 20 µm to 700 µm | - | - | - | 30 | - | - | - | - | - | - | - | - |
| | Crosslinked particle E | 40 µm to 120 µm | - | - | - | - | 30 | - | - | - | - | - | - | - |
| | Crosslinked particle F | 40 µm to 120 µm | - | - | - | - | - | 30 | - | - | - | - | - | - |
| Non-crosslinked partide | Kind | Average particle diameter | | | | | | | | | | | | |
| | Non-crosslinked particle A | - | - | | - | - | - | - | - | - | - | - | - | - |
| Evaluation | Hemostatic property | | 5 | α | 4 | 4 | 4 | 4 | 4 | 5 | 5 | 5 | 3 | 5 |
| | Removability | | 4 | 3 | 4 | 4 | 4 | 4 | 4 | 4 | 4 | 4 | 4 | 3 |
| | Transfer suppressibility | | 5 | 5 | 5 | 5 | 5 | 5 | 3 | 5 | 5 | 5 | 5 | 5 |

**Table 2**

| | Kind | Content of specific structural unit with respect to 100% by mole of specific polysaccharide (% by mole) | Example 13 | Example 14 | Example 15 | Example 16 | Example 17 | Example 18 | Example 19 | Example 20 | Example 21 | Example 22 | Example 23 | Example 24 |
|---|---|---|---|---|---|---|---|---|---|---|---|---|---|---|
| Specific polysaccharide | NHS-carbcoxymethyl dextran A | 14.8% by mole | - | - | 20 | 20 | 20 | 20 | 20 | 5 | 45 | 10 | 30 | 20 |
| | NHS-carboxymethyl pullulan | 6.3% by mole | - | - | - | - | - | - | - | - | - | - | - | - |
| | NHS-carboxymethyl dextrin | 10% by mole | - | - | - | - | - | - | - | - | - | - | - | - |
| | NHS-carboxymethyl dextran B | 21% by mole | - | - | - | - | - | - | - | - | - | - | - | - |
| | NHS-carboxymethyl dextran C | 2.2% by mole | - | - | - | - | - | - | - | - | - | - | - | - |
| | HHS-carboxymethyl dextran D | 15% by mule | - | - | - | - | - | - | - | - | - | - | - | - |
| | NHS-carboxymethyl dextran E | 15% by mole | 20 | - | - | - | - | - | - | - | - | | | |
| | Isocyanated chitosan | - | - | 20 | - | - | - | - | - | - | - | - | - | - |
| Hydrophilic solvent | Kind | Solubility in ion exchange water at 20°C | | | | | | | | | | | | |
| | Vegetable oil A | Less than 20 g/L | 50 | 50 | - | - | - | - | - | 50 | 50 | 75 | 25 | 50 |
| | Vegetable oil B | Less than 20 g/L | - | - | 50 | - | - | - | - | - | - | - | - | - |
| | Paraffin-based solvent | Less than 20 g/L | - | - | - | 50 | - | - | - | - | - | - | - | - |
| | Silicone oil-based solvent A | Less than 20 g/L | - | - | - | - | 50 | - | - | - | - | - | - | - |
| | Silicone oil-based solvent β | Less than 20 g/L | - | - | - | - | - | 50 | - | - | - | - | - | - |
| | Fatty acid ester-based solvent | Less than 20 g/L | - | - | - | - | - | - | 50 | - | - | - | - | - |
| Crosslinked particle | Kind | Average particle diameter | | | | | | | | | | | | |
| | Crosslinked partide A | 40 µm to 60 µm | 30 | 30 | 30 | 30 | 30 | 30 | 30 | 45 | 5 | 15 | 45 | - |
| | Crosslinked particle B | 60 µm | - | - | - | - | - | - | - | - | - | - | - | - |
| | Crosslinked particle C | 50 µm | - | - | - | - | - | - | - | - | - | - | - | - |
| | Crosslinked particle D | 20 µm to 500 µm | - | - | - | - | - | - | - | - | - | - | - | - |
| | Crosslinked particle E | 40 µm to 120 µm | - | - | - | - | - | - | - | - | - | - | - | - |
| | Crosslinked particle F | 40 µm to 120 µm | - | - | - | - | - | - | - | - | - | - | - | - |
| Non-crosslinked particle | Kind | Average particle diameter | | | | | | | | | | | | |
| | Nun-crosslinked particle A | - | - | - | - | - | - | - | - | - | - | - | - | 30 |
| Evaluation | Hemostatic property | | 4 | 3 | 5 | 3 | 3 | 3 | 5 | 3 | 5 | 3 | 3 | 3 |
| | Removability | | 5 | 4 | 4 | 3 | 3 | 3 | 4 | 5 | 3 | 5 | 5 | 3 |
| | Transfer suppressibility | | 5 | 5 | 5 | 5 | 5 | 4 | 5 | 5 | 3 | 5 | 4 | 5 |

**Table 3**

| Specific polysaccharide | Kind | Content of specific structural unit with respect to 100% by mole of specific polysaccharide (% by mole) | Comparative Example 1 | Comparative Example 2 | Comparative Example 3 | Comparative Example 4 | Comparative Example 5 |
|---|---|---|---|---|---|---|---|
| | NHS-carboxymethyl dextran A | 14.8% by mole | - | - | 20 | 20 | 20 |
| Non-specific polysaccharide | Kind | Content of specific structural unit with respect to 100% by mole of specific polysaccharide (% by mole) | | | | | |
| | Dextran 40000 | - | 20 | - | - | - | - |
| Hydrophobic solvent | Kind | Solubility m ion exchange water at 20°C | - | - | - | - | - |
| | Vegetable oil A | Less than 20 g/L | 50 | 50 | - | - | - |
| Hydrophilic solvent | Kind | Solubility in ion exchange water at 20°C | | | | | |
| | Polyethylene glycol | 20 g/L or more | - | - | 50 | - | - |
| | Polyethylene glycol/polypropylene glycol copolymer | 20 g/L or more | - | - | - | 50 | - |
| | Pure water | - | - | - | - | - | 50 |
| Crosslinked particle | Kind | Average particle diameter | - | - | - | - | - |
| | Crosslinked particle A | 40 µm to 60 µm | 30 | 50 | 30 | 30 | 30 |
| Evaluation | Hemostatic property | | 1 | 1 | 2 | 2 | 1 |
| | Removability | | 4 | 4 | 1 | 1 | 5 |
| | Transfer suppressibility | | 5 | 5 | 2 | 2 | 3 |

From Table 1 and Table 2, it can be seen that the hemostatic material composition according to the present disclosure, containing the specific polysaccharide and the hydrophobic solvent, is excellent in safety and hemostatic properties, where the use of thrombin is not necessary.

From Table 3, it can be seen that there is room for improvement in the hemostatic properties of the hemostatic material composition containing the hemostatic material composition which does not contain the specific polysaccharide and containing the hydrophilic solvent.

## Claims

1. A hemostatic material composition comprising:
a polysaccharide having a reactive group that reacts with a protein to form a crosslinking structure; and
a hydrophobic solvent.

2. The hemostatic material composition according to claim 1,
wherein the reactive group is one or more groups selected from the group consisting of -CON(COCH₂)₂, an N-hydroxysuccinimide ester group, an imide ester group, an aldehyde group, a carboxy group, an isocyanate group, a maleimide group, and a haloacetyl group.

3. The hemostatic material composition according to claim 1 or 2,
wherein the reactive group is an N-hydroxysuccinimide ester group.

4. The hemostatic material composition according to any one of claims 1 to 3,
wherein the polysaccharide includes a structure derived from one or more polysaccharides selected from the group consisting of cellulose, dextran, dextrin, pullulan, hyaluronic acid, alginic acid, xanthan gum, chitin, chitosan, and a modified product thereof.

5. The hemostatic material composition according to any one of claims 1 to 4,
wherein the polysaccharide contains a constitutional unit derived from a monosaccharide having the reactive group, and
a content of a constitutional unit derived from the monosaccharide having the reactive group is 1% by mole to 40% by mole with respect to 100% by mole of the polysaccharide.

6. The hemostatic material composition according to claim 5,
wherein the constitutional unit derived from the monosaccharide having the reactive group is represented by General Formula (1), 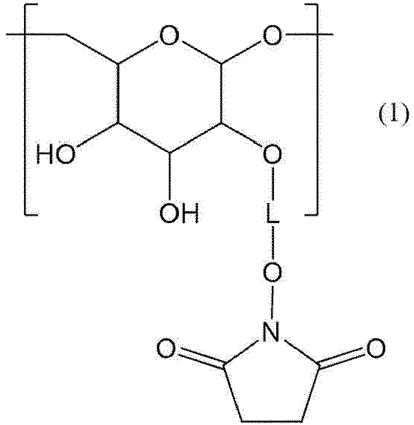
in General Formula (1), L is a group represented by the following formula, 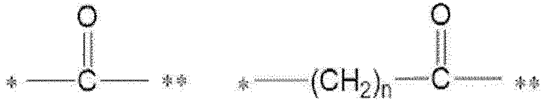 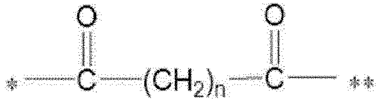 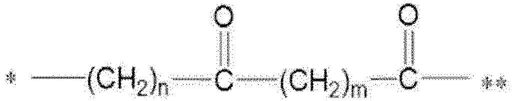
in the formula, n and m represent an integer of 1 to 3, * represents a site linked to an oxygen atom on a tetrahydrofuran side, and ** represents a site linked to an oxygen atom on an N-hydroxysuccinimide ester group side.

7. The hemostatic material composition according to any one of claims 1 to 6,
wherein a weight-average molecular weight of the polysaccharide is 2,000 to 2,500,000.

8. The hemostatic material composition according to any one of claims 1 to 7,
wherein the hydrophobic solvent is one or more selected from the group consisting of a vegetable oil, a paraffin-based solvent, an olefin-based solvent, a silicone oil-based solvent, a fatty acid ester-based solvent, an alkyl halide-based solvent, and a terpene-based solvent.

9. The hemostatic material composition according to any one of claims 1 to 8,
wherein a kinematic viscosity of the hydrophobic solvent at 25°C is 1 mm²/sec to 1,000 mm²/sec.

10. The hemostatic material composition according to any one of claims 1 to 9,
wherein a content of the hydrophobic solvent is 30% by mass to 70% by mass with respect to a total mass of the hemostatic material composition.

11. The hemostatic material composition according to any one of claims 1 to 10, further comprising a crosslinked particle.

12. The hemostatic material composition according to claim 11,
wherein the crosslinked particle contains one or more compounds selected from the group consisting of a crosslinked protein and a crosslinked polysaccharide.

13. The hemostatic material composition according to claim 12,
wherein the crosslinked particle contains the crosslinked polysaccharide, and
the crosslinked polysaccharide is a crosslinked dextran.

14. The hemostatic material composition according to claim 13,
wherein the crosslinked dextran is a crosslinked dextran modified with an aminoalkyl group.

15. The hemostatic material composition according to claim 14,
wherein the aminoalkyl group is a diethylaminoethyl group.

16. The hemostatic material composition according to any one of claims 11 to 15,
wherein an average particle diameter of the crosslinked particles is 20 µm to 500 µm.

## Patentansprüche

1. Hämostatische Materialzusammensetzung umfassend:
ein Polysaccharid mit einer reaktiven Gruppe, die mit einem Protein reagiert, um eine vernetzende Struktur zu bilden; und
ein hydrophobes Lösungsmittel.

2. Hämostatische Materialzusammensetzung nach Anspruch 1,
wobei die reaktive Gruppe eine oder mehrere Gruppen, ausgewählt aus der Gruppe bestehend aus -CON(COCH₂)₂, einer N-Hydroxysuccinimidestergruppe, einer Imidestergruppe, einer Aldehydgruppe, einer Carboxylgruppe, einer Isocyanatgruppe, einer Maleimidgruppe und einer Halacetylgruppe ist.

3. Hämostatische Materialzusammensetzung nach Anspruch 1 oder 2,
wobei die reaktive Gruppe eine N-Hydroxysuccinimidestergruppe ist.

4. Hämostatische Materialzusammensetzung nach einem der Ansprüche 1 bis 3,
wobei das Polysaccharid eine Struktur enthält, die sich von einem oder mehreren Polysacchariden, ausgewählt aus der Gruppe bestehend aus Cellulose, Dextran, Dextrin, Pullulan, Hyaluronsäure, Alginsäure, Xanthangummi, Chitin, Chitosan und einem modifizierten Produkt davon ableitet.

5. Hämostatische Materialzusammensetzung nach einem der Ansprüche 1 bis 4,
wobei das Polysaccharid eine von einem Monosaccharid mit einer reaktiven Gruppe abgeleitete konstitutionelle Einheit enthält und
ein Gehalt einer konstitutionellen Einheit, die sich von dem Monosaccharid mit der reaktiven Gruppe ableitet, 1 Mol% bis 40 Mol%, bezogen auf 100 Mol% des Polysaccharids beträgt.

6. Hämostatische Materialzusammensetzung nach Anspruch 5,
wobei die von dem Monosaccharid mit der reaktiven Gruppe abgeleitete konstitutionelle Einheit durch die allgemeine Formel (1) dargestellt ist, 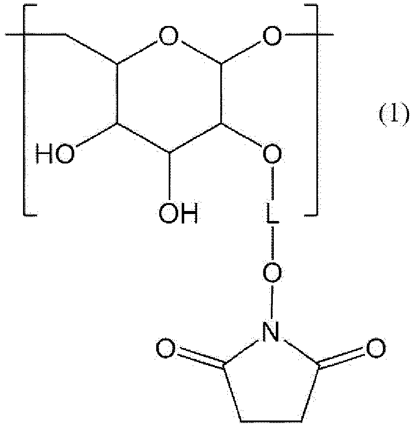
wobei in der allgemeinen Formel (1) L eine durch die folgende Formel dargestellte Gruppe darstellt, 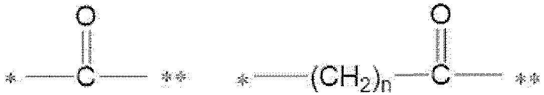 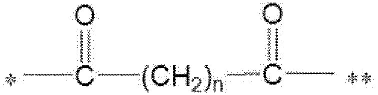 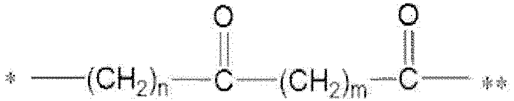
wobei in der Formel n und m eine ganze Zahl von 1 bis 3 darstellen, * eine Stelle darstellt, die an ein Sauerstoffatom an einer Tetrahydrofuranseite gebunden ist und ** eine Stelle darstellt, die an ein Sauerstoff an einer N-Hydroxysuccinimidestergruppenseite gebunden ist.

7. Hämostatische Materialzusammensetzung nach einem der Ansprüche 1 bis 6,
wobei ein gewichtsgemitteltes Molekulargewicht des Polysaccharids 2000 bis 2500000 beträgt.

8. Hämostatische Materialzusammensetzung nach einem der Ansprüche 1 bis 7,
wobei das hydrophobe Lösungsmittel eines oder mehrere, ausgewählt aus der Gruppe bestehend aus einem pflanzlichen Öl, einem Paraffin-basierten Lösungsmittel, einem Olefinbasierten Lösungsmittel, einem Silikonöl-basierten Lösungsmittel, einem Fettsäureesterbasierten Lösungsmittel, einem Alkylhalid-basierten Lösungsmittel und einem Terpenbasierten Lösungsmittel ist.

9. Hämostatische Materialzusammensetzung nach einem der Ansprüche 1 bis 8,
wobei eine kinematische Viskosität des hydrophoben Lösungsmittels bei 25°C 1 mm²/sec bis 1,000 mm²/sec beträgt.

10. Hämostatische Materialzusammensetzung nach einem der Ansprüche 1 bis 9,
wobei ein Gehalt an dem hydrophoben Lösungsmittel 30 Masse% bis 70 Masse%, bezogen auf die Gesamtmasse der hämostatischen Materialzusammensetzung beträgt.

11. Hämostatische Materialzusammensetzung nach einem der Ansprüche 1 bis 10, ferner umfassend einen vernetzten Partikel.

12. Hämostatische Materialzusammensetzung nach Anspruch 11,
wobei der vernetzte Partikel eine oder mehrere Verbindungen, ausgewählt aus der Gruppe bestehend aus einem vernetzten Protein und einem vernetzten Polysaccharid, enthält.

13. Hämostatische Materialzusammensetzung nach Anspruch 12,
wobei der vernetzte Partikel das vernetzte Polysaccharid enthält und
das vernetzte Polysaccharid ein vernetztes Dextran ist.

14. Hämostatische Materialzusammensetzung nach Anspruch 13,
wobei das vernetzte Dextran ein mit einer Aminoalkylgruppe modifiziertes vernetztes Dextran, ist.

15. Hämostatische Materialzusammensetzung nach Anspruch 14,
wobei die Aminoalkylgruppe eine Diethylaminoethylgruppe ist.

16. Hämostatische Materialzusammensetzung nach einem der Ansprüche 11 bis 15,
wobei ein durchschnittlicher Partikeldurchmesser der vernetzten Partikel 20 µm bis 500 µm beträgt.

## Revendications

1. Composition de matériau hémostatique, comprenant :
un polysaccharide présentant un groupe réactif, lequel réagit avec une protéine pour former une structure de réticulation, et
un solvant hydrophobe.

2. Composition de matériau hémostatique selon la revendication 1,
dans laquelle le groupe réactif est un ou plusieurs groupes sélectionnés parmi le groupe consistant en -CON(COCH₂)₂, un groupe ester N-hydroxysuccinimide, un groupe ester imide, un groupe aldéhyde, un groupe carboxy, un groupe isocyanate, un groupe maléimide, et un groupe haloacétyle.

3. Composition de matériau hémostatique selon la revendication 1 ou 2,
dans laquelle le groupe réactif est un groupe ester N-hydroxysuccinimide.

4. Composition de matériau hémostatique selon l'une quelconque des revendications 1 à 3,
dans laquelle le polysaccharide inclut une structure dérivée d'un ou de plusieurs polysaccharides sélectionnés parmi le groupe consistant en la cellulose, la dextrane, la dextrine, le pullulane, l'acide hyaluronique, l'acide alginique, la gomme de xanthane, la chitine, le chitosane, et un produit modifié de ceux-ci.

5. Composition de matériau hémostatique selon l'une quelconque des revendications 1 à 4,
dans laquelle le polysaccharide contient un motif constitutif dérivé d'un monosaccharide présentant le groupe réactif, et
une teneur d'un motif constitutif dérivé du monosaccharide présentant le groupe réactif s'étend de 1% en moles à 40 % en moles par rapport à 100 % en moles du polysaccharide.

6. Composition de matériau hémostatique selon la revendication 5,
dans laquelle le motif constitutif dérivé du monosaccharide présentant le groupe réactif est représenté par la formule générale (1), 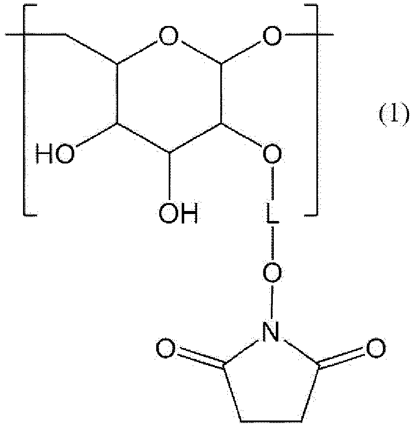
dans la formule générale (1), L est un groupe représenté par la formule suivante : 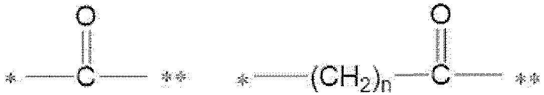 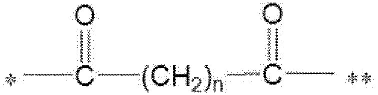 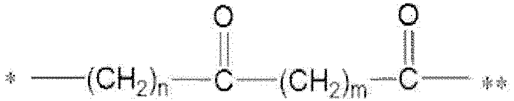
dans la formule, n et m représentent un entier allant de 1 à 3 ; * représente un site lié sur un atome d'oxygène sur un côté de tétrahydrofurane, et ** représente un site lié sur un atome d'oxygène sur un côté de groupe ester N-hydroxysuccinimide.

7. Composition de matériau hémostatique selon l'une quelconque des revendications 1 à 6,
dans laquelle un poids moléculaire en poids moyen du polysaccharide s'étend de 2000 à 2 500 000.

8. Composition de matériau hémostatique selon l'une quelconque des revendications 1 à 7,
dans laquelle le solvant hydrophobe est un ou plusieurs éléments sélectionnés parmi le groupe consistant en une huile végétale, un solvant à base de paraffine, un solvant à base d'oléfine, un solvant à base de silicone, un solvant à base d'ester d'acide gras, un solvant à base d'halogénure d'alkyle, et un solvant à base de terpène.

9. Composition de matériau hémostatique selon l'une quelconque des revendications 1 à 8,
dans laquelle une viscosité cinématique du solvant hydrophobe à 25 °C s'étend de 1 mm²/s à 1000 mm²/s.

10. Composition de matériau hémostatique selon l'une quelconque des revendications 1 à 9,
dans laquelle une teneur en solvant hydrophobe s'étend de 30% en masse à 70 % en masse par rapport à une masse totale de la composition de matériau hémostatique.

11. Composition de matériau hémostatique selon l'une quelconque des revendications 1 à 10, comprenant en outre une particule réticulée.

12. Composition de matériau hémostatique selon la revendication 11,
dans laquelle la particule réticulée contient un ou plusieurs composés sélectionnés parmi le groupe consistant en une protéine réticulée et un polysaccharide réticulé.

13. Composition de matériau hémostatique selon la revendication 12,
dans laquelle la particule réticulée contient le polysaccharide réticulé, et
le polysaccharide réticulé est de la dextrane réticulée.

14. Composition de matériau hémostatique selon la revendication 13,
dans laquelle la dextrane réticulée est une dextrane réticulée modifiée avec un groupe aminoalkyle.

15. Composition de matériau hémostatique selon la revendication 14,
dans laquelle le groupe aminoalkyle est un groupe diéthylaminoéthyle.

16. Composition de matériau hémostatique selon l'une quelconque des revendications 11 à 15,
dans laquelle un diamètre de particule moyen des particules réticulées s'étend de 20 µm à 500 µm.
